## Europäisches Patentamt

(19) **European Patent Office**

**Office européen des brevets**

(11) Veröffentlichungsnummer: **0 010 223**
**B1**

(12) # EUROPÄISCHE PATENTSCHRIFT

(45) Veröffentlichungstag der Patentschrift:
**16.09.81**

(51) Int. Cl.³: **C 07 C 7/04,** B 01 D 3/00

(21) Anmeldenummer: **79103768.2**

(22) Anmeldetag: **03.10.79**

(54) **Verfahren zum Zerlegen eines Gasgemisches.**

(30) Priorität: **09.10.78 DE 2843982**

(43) Veröffentlichungstag der Anmeldung:
**30.04.80 Patentblatt 80/9**

(45) Bekanntmachung des Hinweises auf die Patenterteilung:
**16.09.81 Patentblatt 81/37**

(84) Benannte Vertragsstaaten:
**AT BE DE FR GB IT NL**

(56) Entgegenhaltungen:
**DE-A1-2 552 140**
**DE-A1-2 608 404**

(73) Patentinhaber: **Linde Aktiengesellschaft,**
**Abraham-Lincoln-Strasse 21, D-6200 Wiesbaden (DE)**

(72) Erfinder: **Mikulla, Klaus-Dieter, Brauneckweg 6,**
**D-8192 Geretsried 1 (DE)**

(74) Vertreter: **Schaefer, Gerhard, Dr., Linde**
**Aktiengesellschaft Zentrale Patentabteilung,**
**D-8023 Höllriegelskreuth (DE)**

Verfahren zum Zerlegen eines Gasgemisches

Die Erfindung betrifft ein Verfahren zum Zerlegen eines im wesentlichen aus Kohlenwasserstoffen bestehenden Gasgemisches, bei dem durch ein- oder mehrstufige partielle Kondensation Teile des Gasgemisches verflüssigt werden, wobei die dabei gebildeten flüssigen Fraktionen in einer ersten Rektifiziersäule weiter zerlegt werden und wobei nach der letzten Stufe der partiellen Kondensation die schwer flüchtigen Bestandteile aus der verbliebenen gasförmigen Fraktion durch Rektifikation in einer zweiten Rektifiziersäule entfernt werden.

Derartige Verfahren finden beispielsweise bei der Gewinnung von Äthylen aus den Produktgasen einer Anlage zur Spaltung von Kohlenwasserstoffen Anwendung. Dabei wird üblicherweise das aus der Spaltung kommende Gasgemisch verdichtet und durch Kühlung partiell kondensiert. Die dabei gewonnenen Fraktionen werden in einem bekannten Verfahren in eine Rektifikationssäule eingespeist, in der eine scharfe Trennung in zwei Fraktionen stattfindet, von denen die erste $C_2$-Kohlenwasserstoffe und leichter siedende Bestandteile und die zweite $C_3$-Kohlenwasserstoff und schwerer siedende Bestandteile enthält. Zur Kondensation der leichter siedenden Fraktion am Kopf der Rektifiziersäule werden grosse Mengen an Kälte auf relativ niedrigem Temperaturniveau benötigt, während am Sumpf mit Dampf geheizt wird. Dieses bekannte Verfahren ist aus diesem Grunde mit einem grossen Energiebedarf verbunden.

Aus der DE-OS 26 08 404 ist bereits ein Verfahren zur Gaszerlegung bekannt, bei dem der Energieaufwand dadurch verringert wird, dass die Rektifikation auf zwei getrennte Säulen verteilt wird, die in unterschiedlichen Temperaturbereichen und mit relativ kleinen Temperaturdifferenzen zwischen Kopf und Sumpf arbeiten. Ferner ist aus der DE-OS 25 52 140 ein Verfahren zur Abtrennung von $C_{2+}$-Kohlenwasserstoffen aus Gasgemischen bekannt, bei dem das Ausgangsgas in einem Dephlegmator gekühlt und das dabei anfallende Kondensat in einer Rektifikationssäule in einen $C_{1-}$-Strom und einen $C_{2+}$-Strom zerlegt wird. Im Gegensatz zur DE-OS 26 08 404 und zu einem Verfahren der eingangs genannten Art arbeitet dieses Verfahren nur mit einer Rektifikationssäule.

Der Erfindung liegt die Aufgabe zugrunde, ein solches Verfahren so abzuändern, dass weitere Energieeinsparungen möglich sind.

Diese Aufgabe wird dadurch gelöst, dass von den flüssigen Fraktionen vor deren Einspeisung in die erste Rektifiziersäule die am leichtesten flüchtigen Bestandteile abgestrippt werden.

Durch die erfindungsgemässe Verfahrensführung werden die am leichtesten flüchtigen Bestandteile des Gasgemisches praktisch vollständig von der ersten Rektifiziersäule ferngehalten. Dadurch wird der Taupunkt des im Kopf der ersten Rektifiziersäule zu kondensierenden Gemisches in Richtung auf höhere Temperaturen verschoben, d.h. der Kopf der Zerlegungssäule kann mit Kälte höherer Temperatur und somit billigerer Kälte gekühlt werden.

Da beim erfindungsgemässen Verfahren im Gegensatz zum bekannten Verfahren der DE-OS 26 08 404 keine leichten Bestandteile mehr in die erste Rektifiziersäule gelangen, kann diese Säule unter einem niedrigeren Druckniveau betrieben werden. Dies führt nicht nur zu einer Einsparung von Verdichterenergie, sondern ermöglicht darüber hinaus auch ein günstigeres, d.h. niedrigeres Rücklaufverhältnis in der ersten Rektifiziersäule, weil sich die Gleichgewichtsbedingungen des Gasgemisches bei abnehmendem Druck verbessern.

In einer vorteilhaften Ausgestaltung des erfindungsgemässen Verfahrens ist die zweite Rektifiziersäule in ihrem unteren Teil ebenfalls mit einem Strippteil ausgestattet. Das Gasgemisch in der zweiten Rektifiziersäule besteht im wesentlichen aus leicht flüchtigen Komponenten und enthält nur noch verhältnismässig wenig schwer flüchtige Bestandteile. Da aber bei der Rektifikation nicht nur die schwer flüchtigen Bestandteile im Sumpf anfallen, sondern im Gleichgewicht damit auch noch einige leicht flüchtige Komponenten, ist es günstig, diese hier ebenso abzustrippen wie bei den Kondensaten der vorhergegangenen partiellen Kondensationen. Dabei bildet sich dann ein Sumpfprodukt, das in günstiger Weiterbildung des Verfahrens ebenfalls der ersten Rektifiziersäule an einer geeigneten Stelle zur weiteren Zerlegung aufgegeben wird.

Günstig ist es weiterhin, die aus den flüssigen Fraktionen abgestrippten Bestandteile wieder den jeweiligen gasförmigen Fraktionen zuzuführen. Dabei ist es in vielen Fällen möglich, auf einen besonderen Abscheider für die flüssigen Fraktionen zu verzichten und stattdessen nur eine Strippsäule vorzusehen, in deren Sumpf das von den leicht flüchtigen Bestandteilen befreite Kondensat und an deren Kopf die übrigen Bestandteile anfallen.

Ein besonderer Vorteil des erfindungsgemässen Verfahrens zeigt sich in einer weiteren Ausgestaltung, bei der das Kopfprodukt der ersten Rektifiziersäule vollständig verflüssigt wird, worauf ein Teil als Rücklaufflüssigkeit für die erste Rektifiziersäule und der Rest nach weiterer Abkühlung als Rücklauf für die zweite Rektifiziersäule verwendet wird. Da sich nämlich gezeigt hat, dass wegen des geringen Bedarfs an Rücklaufflüssigkeit in der ersten Rektifiziersäule die Kopfproduktmenge in vielen Fällen ausreicht, auch den Rücklaufbedarf für die zweite Rektifiziersäule zu decken, kann auf eine besondere Kondensationsstufe am Kopf der zweiten Rektifiziersäule verzichtet werden. Diese Verfahrensführung bietet darüber hinaus den Vorteil, dass das Kopfprodukt der ersten Rektifiziersäule noch in die zweite Rektifiziersäule geführt wird, wodurch die Anforderungen an die Trennung in der ersten Rektifizersäule herabgesetzt werden können.

Das erfindungsgemässe Verfahren hat ein sehr weites Feld von Anwendungsmöglichkeiten. Bei der Zerlegung der Spaltgase einer Äthylenanlage kann das Verfahren beispielsweise bei einer $C_2/C_3$-Trennung verwendet werden, wobei aus den Kondensaten $C_1$-Kohlenwasserstoffe und tiefer siedende Bestandteile abgestrippt werden. Eine andere Anwendungsmöglichkeit innerhalb einer Äthylenanlage besteht bei der $C_1/C_2$-Trennung, bei der als tiefer siedende Komponente Wasserstoff aus den Kondensaten abgestrippt werden kann. Auch die Aufbereitung von Erdgasen ist ein mögliches Anwendungsgebiet. Dabei können beispielsweise bei der partiellen Kondensation von $C_2$- bis $C_7$-Kohlenwasserstoffen die Kondensate $C_{1-}$ freigemacht werden.

Nachfolgend wird das erfindungsgemässe Verfahren anhand einiger in den Figuren schematisch dargestellten Ausführungsbeispiele näher erläutert.

Die Figuren zeigen ein Verfahren, das geeignet ist, im Rahmen einer Äthylenanlage die $C_2/C_3$-Trennung durchzuführen. Dabei sind für das in Figur 1 dargestellte Verfahren drei Strippkolonnen vor der Rektifikation vorgesehen, während im Verfahren der Figur 2 nur zwei und im Verfahren der Figur 3 nur eine Strippkolonne vorgesehen ist.

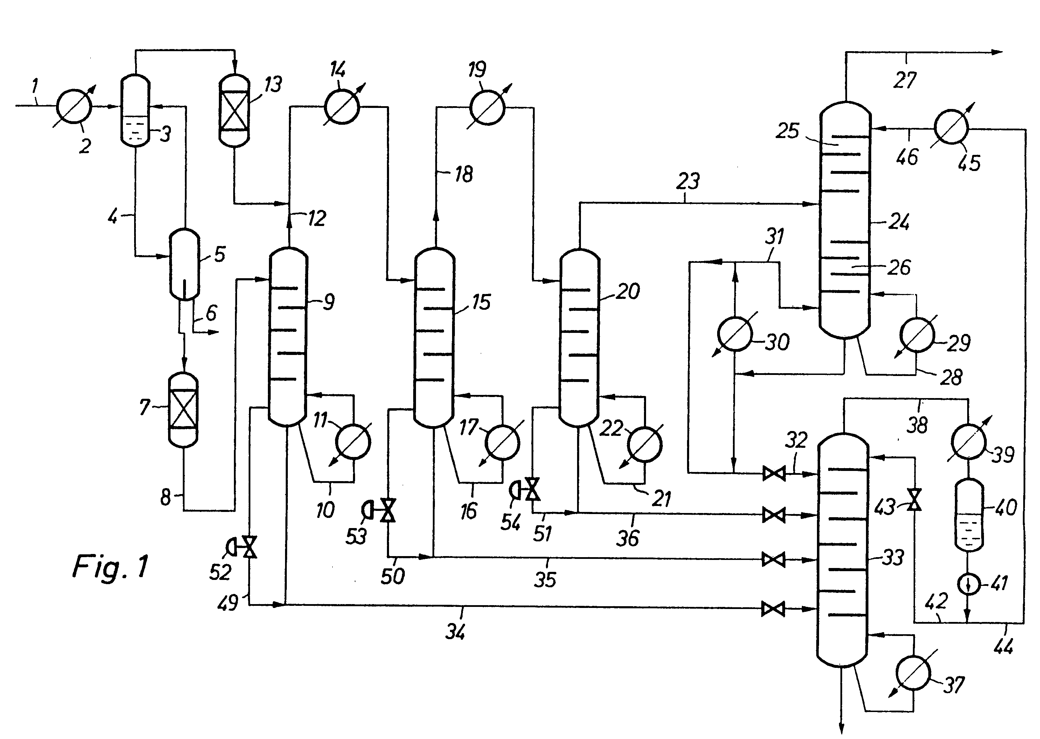

Bei dem Verfahren gemäss Figur 1 treten über Leitung 1 3789 Nm³/H Kondensat und Rohgas in einer Menge von 115 058 Nm³/h unter einem Druck von 34,6 bar und einer Temperatur von 305 K in die Anlage ein. Im Wärmetauscher 2, der mehrstufig ausgebildet sein kann, wird es auf eine Temperatur von 288 K abgekühlt. Als Kühlmittel werden dabei bereits teilweise wieder angewärmte Produktströme aus dem Tieftemperaturteil der Anlage verwendet. Bei dieser ersten Kühlung werden dem Rohgas 1,8 Gcal/h (7,5 GJ/h) Wärme entzogen. Dabei kondensiert ein Teil der schweren Komponenten des Gasgemisches, wobei im Gleichgewicht damit auch einige leichte Komponenten in Lösung gehen. Das Gemisch wird im Abscheider 3 einer Phasentrennung unterzogen. Die flüssige Phase wird über Leitung 4 zunächst in einen Wasserabscheider 5 geführt, aus dem das abgetrennte Wasser über Leitung 6 abgezogen wird. Anschliessend werden im Trockner 7 noch die verbliebenen Reste von Wasser entfernt, wonach 8337 Nm³/h Kondensat über Leitung 8 in den oberen Teil einer Strippkolonne 9 geführt werden.

Die Strippkolonne 9, deren Druck am Kopf 34,1 bar und im Sumpf 34,2 bar beträgt und deren theoretische Bodenzahl 6 ist, wird bei Temperaturen zwischen 296 K am Kopf und 354 K im Sumpf und bei einem Rücklaufverhältnis von 3,35 (Flüssigkeit/Dampf) betrieben.

Ein Teil des Sumpfprodukts wird über Leitung 10 abgezogen, im Wärmetauscher 11 gegen Niederdruckdampf erwärmt und dann wieder in den Sumpf eingeführt. Für die Sumpfbeheizung wird eine Wärmemenge von 0,72 Gcal (3.0 GJ/h) benötigt, um 6485 Nm³/h $C_{1-}$-freies Sumpfprodukt und 1852 Nm³/h Kopfprodukt zu erhalten.

Das Kopfprodukt wird über Leitung 12 abgezogen und mit der gasförmigen Fraktion aus dem Abscheider 3 nach dessen Trocknung 13 vereinigt. Es wird dann im Wärmetauscher 14 gegen Produktgase auf eine Temperatur von 258 K abgekühlt, wobei wiederum ein Teil des Gasgemisches kondensiert. Bei diesem Wärmetausch werden dem Gasgemisch 3,6 Gcal/h (15,1 GJ/h) Wärme entzogen.

Das Gemisch wird danach in eine zweite Strippkolonne 15 geführt, in der wiederum ein $C_{1-}$-freies Kondensat als Sumpfprodukt abgetrennt wird. Um dies zu erreichen, ist die unter einem Druck von 33,7 bar am Kopf und 33,8 bar im Sumpf arbeitende Säule mit neun theoretischen Böden ausgerüstet und wird bei Temperaturen von 264 K am Kopf und 311 K im Sumpf betrieben. Das Rücklaufverhältnis liegt bei 3,61.

Über Leitung 16 wird ein Teil des Sumpfprodukts einem Wärmetauscher 17 zugeführt, über den dem Kolonnensumpf 0,93 Gcal/h (3,9 GJ/h) an Wärme zugeführt werden. Es bilden sich im Sumpf 11 969 Nm³/h $C_{1-}$-freies Produkt, während 3 420 Nm³/h leichte Bestandteile aus dem Kondensat der zweiten Kühlstufe abgestrippt werden. Wegen des niedrigen Temperaturniveaus von 311 K im Sumpf der Strippkolonne 15 kann die Beheizung des Wärmetauschers 17 mit Waschwasser erfolgen, das bei der Quenchung der heissen Spaltgase verwendet wird. Es fällt bei einem Temperaturniveau von etwa 350 K an und kann nicht mehr für eine Dampferzeugung verwendet werden. Durch die Ausnutzung dieses Wärmepotentials, das im allgemeinen grösstenteils als Abwärme ungenutzt an die Umgebung abgeführt wird, verbessert sich die Wirtschaftlichkeit des Verfahrens erheblich.

Die gasförmig verbliebenen Bestandteile des Gasgemisches gelangen anschliessend über Leitung 18 in eine dritte Kondensationsstufe, in der eine Wärmemenge von 2,6 Gcal/h (10,5 GJ/h) aus dem Gasgemisch abgezogen wird. Dabei wird das Gasgemisch im Wärmetauscher 19 auf eine Temperatur von 239 K abgekühlt. Danach wird es in eine dritte Strippkolonne 20 geführt, in der wiederum ein $C_{1-}$-freies Kondensat im Sumpf abgetrennt wird. Die Kolonne 20 arbeitet unter einem Druck von 33,6 bar am Kopf und 33,7 bar im Sumpf. Sie enthält sieben theoretische Böden und wird bei Temperaturen zwischen 254 K im Kopf und 292 K im Sumpf bei einem Rücklaufverhältnis von 2,36 betrieben.

Wie in den Kolonnen 9 und 15, so wird auch hier über Leitung 21 ein Teil des Sumpfprodukts durch einen Erhitzer 22 geleitet, in dem 1,0 Gcal/h (4,2 GJ/h) Wärme aufgenommen werden, um 10 352 Nm³/h $C_{1-}$-freies Sumpfprodukt zu erhalten. Dabei werden 5 421 Nm³/h leichte Bestandteile aus dem Kondensat der dritten Kühlstufe abgetrennt.

Der Wärmetauscher 22 wird ebenso wie der Wärmetauscher 17 mit billiger Abwärme aus dem Waschwasser der Quenchung beheizt.

Die nicht kondensierten Bestandteile des Gasgemisches, die nur noch 2,4 Mol-% $C_3$-Kohlenwasserstoffe und schwerere Komponenten enthalten, werden über Leitung 23 in die Rektifiziersäule 24 (2. Rektifiziersäule) geführt. Diese Rektifiziersäule 24 ist mit einem Rektifizierteil 25 im oberen Bereich und einem Strippteil 26 im unteren Bereich ausgerüstet, zwischen denen das Gasgemisch eingespeist wird. Im Rektifizierteil 25 werden die im Gasgemisch noch verbliebenen Komponenten, die schwerer als $C_2$-Kohlenwasserstoffe sind, abgetrennt, so dass als Kopfprodukt über Leitung 27 ein Gemisch von $C_2$-Kohlenwasserstoffen und leichteren Bestandteilen abgezogen werden kann, das praktisch keine $C_3$-Kohlenwasserstoffe und schwerere Komponenten mehr enthält.

Die $C_1$-Kohlenwasserstoffe und die noch leichter siedenden Komponenten, die bei der Rektifikation in Lösung gegangen sind, werden im Strippteil 26 wieder abgestrippt. Dazu wird, entsprechend den Kolonnen 9, 15 und 20, wieder ein Teil des Sumpfprodukts erwärmt und in den Sumpf zurückgeführt. Ein Teil des Sumpfprodukts wird dazu über Leitung 28 durch einen Wärmetauscher 29 geführt, der ebenfalls wieder mit Waschwasser beheizt werden kann, während ausserdem ein anderer Teil des Sumpfprodukts in einem Wärmetauscher 30 erwärmt und über Leitung 31 zurückgeführt wird. Dieser Wärmetauscher 30 kann mit Rohgas beheizt werden, er kann beispielsweise einen Querschnitt des Wärmetauschers 2 bilden. Darüber hinaus kann der Sumpf der Rektifiziersäule 24 auch mit Kühlmitteln, beispielsweise Propylen, geeigneter Temperatur beheizt werden.

Die Rektifiziersäule 24 wird unter einem Druck von 33,4 bar im Kopf und 33,6 bar im Sumpf betrieben. Sie ist mit dreizehn theoretischen Böden ausgerüstet und arbeitet bei Temperaturen zwischen 234 K am Kopf und 286 K im Sumpf. Bei einem Rücklaufverhältnis von 0,20 fallen dabei 7 775 Nm³/h $C_1$-freies Kondensat an, während als Kopfprodukt 100 858 Nm³/h $C_{3+}$-freies Gasgemisch abgezogen werden. Der Sumpf der Rektifiziersäule 24 wird mit 1,0 Gcal/h (4,2 GJ/h) beheizt.

Das abgezogene $C_1$-freie Sumpfprodukt der Rektifiziersäule 24 wird entspannt und über Leitung 32 in den oberen Bereich der Rektifiziersäule 33 geführt. Ausserdem werden die $C_1$-freien Sumpfprodukte der Kolonnen 9, 15 und 20 über die Leitungen 34, 35 bzw. 36 getrennt voneinander nach Entspannung in die Rektifiziersäule 33 eingeführt. Das Kondensat mit den schwersten Komponenten aus Kolonne 9 wird dabei an einer tieferen Stelle in die Rektifiziersäule 33 eingeführt als die leichteren Kondensate aus den Kolonnen 15 und 20, wobei die geeigneten Einspeisestellen aus dem in der Rektifiziersäule 33 vorliegenden Gleichgewicht und den Kondensatzusammensetzungen in den Leitungen 32, 34, 35 und 36 zu ermitteln sind. Um die Trennung in der Rektifiziersäule 33 unter besonders günstigen Bedingungen durchführen zu können, wird im Sumpf der Strippkolonnen 9, 15 und 20 ein geringer Dampfüberschuss gebildet, der über die Leitungen 49, 50 bzw. 51 abgezogen und mit den Kondensaten in die Rektifiziersäule eingespeist wird. Die zugeführte Dampfmenge kann durch Ventile 52, 53 und 54 geregelt werden.

Da der Rektifiziersäule 33 als leichteste Komponente $C_2$-Kohlenwasserstoffe aufgegeben werden, ist die Trennung in eine $C_2$- und eine $C_{3+}$-Fraktion mit geringem Energieaufwand durchführbar. Die Trennung erfolgt bei einem Säulendruck von 26 bar am Kopf und von 26,3 bar im Sumpf, wobei einunddreissig theoretische Böden vorgesehen sind, und wobei Temperaturen zwischen 260 K am Kopf und 360 K im Sumpf herrschen. Zur Aufrechterhaltung der Sumpftemperatur werden im Wärmetauscher 37 3,6 Gcal/h (15,1 GJ/h) zugeführt. Bei einem Rücklaufverhältnis von 0,46 fallen als Sumpfprodukt 17 632 Nm³/h einer $C_2$-freien Fraktion an, während 18 948 Nm³/h Kopfprodukt als praktisch $C_{3+}$-freie Fraktion über Leitung 38 abgezogen werden.

Dieses Kopfprodukt wird im Wärmetauscher 39 gegen ein Kühlmittel bei 253 K vollständig verflüssigt, wobei 3,2 Gcal/h (13,4 GJ/h) an das Kühlmittel abgegeben werden. Als Kühlmittel kann beispielsweise Propylen verwendet werden. Das Kondensat wird im Behälter 40 aufgefangen.

Das kondensierte Kopfprodukt wird anschliessend durch die Pumpe 41 auf den Druck der Rektifiziersäule 24 verdichtet. Danach wird über Leitung 42 ein Teil des Kondensats abgezweigt, um nach Entspannung bei 43 wieder in die Rektifiziersäule 33 als Rücklaufflüssigkeit eingeführt zu werden. Der übrige Teil des Kondensats wird über Leitung 44 durch einen Wärmetauscher 45 geführt, in dem es auf 233 K unterkühlt wird. Dabei werden 0,34 Gcal/h (1,4 GJ/h) Wärme an ein Kältemittel, beispielsweise Propylen, übertragen. Die unterkühlte Flüssigkeit wird anschliessend über Leitung 46 der Rektifiziersäule 24 als Rücklaufflüssigkeit aufgegeben.

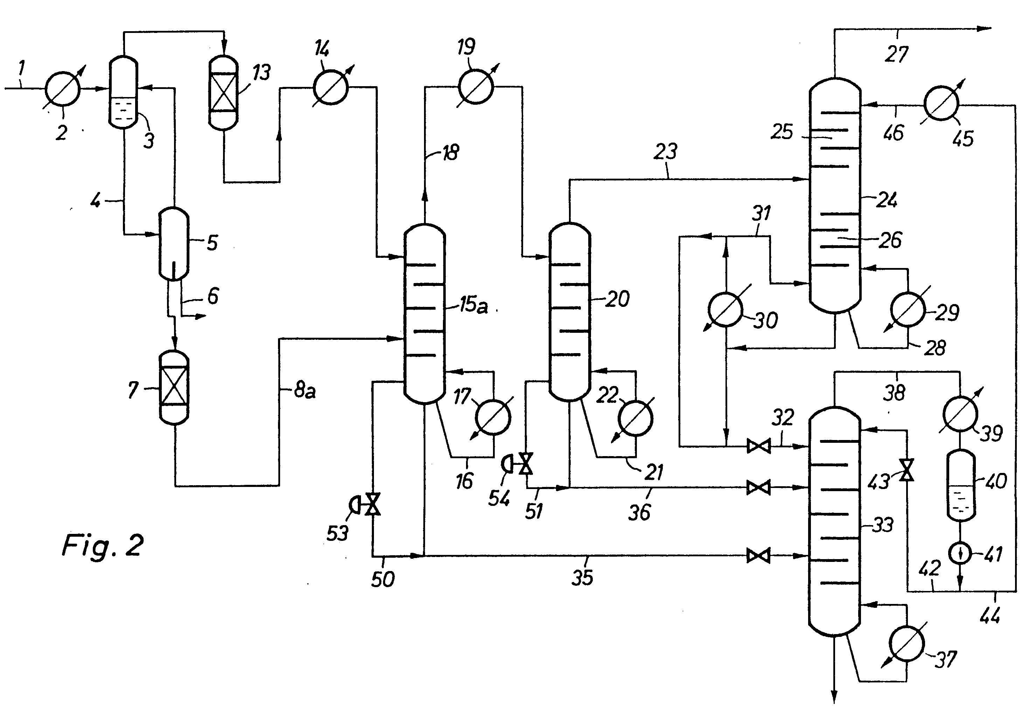

Das in der Figur 2 dargestellte Verfahren unterscheidet sich von dem der Figur 1 im wesentlichen dadurch, das hier statt drei nur zwei Strippkolonnen 15a und 20 vorgesehen sind. Das aus dem Trockner 7 austretende Kondensat wird hier nicht in eine eigene Strippkolonne geführt, sondern gelangt über Leitung 8a in den mittleren Bereich der Strippkolonne 15a, deren oberer Bereich mit dem im Wärmetauscher 14 unter Entzug von 3,5 Gcal/h (14,6 GJ/h) teilweise kondensierten Gasgemisch gespeist wird. Bei dieser Verfahrensführung wird die Kolonne 15a unter anderen Bedingungen betrieben als im Beispiel der Figur 1. Sie ist nunmehr mit elf theoretischen Böden ausgestattet und arbeitet bei Temperaturen zwischen 265 K am Kopf und 323 K im Sumpf. Aus dem Sumpf werden dabei 18 426 Nm³/h $C_1$-freies Kondensat abgezogen, während aus den zugeführten Kondensaten 4 850 Nm³/h leichte Bestandteile abgestrippt werden. Der Sumpfbeheizung werden stündlich 1,5 Gcal (6,3 GJ) aus Waschwasser über den Wärmetauscher 17 zugeführt.

Dem Kopfprodukt aus Kolonne 15a werden im Wärmetauscher 19 stündlich 1,7 Gcal (7,1 GJ) Wärme entzogen, wobei es sich auf 254 K abkühlt und teilweise kondensiert. In der Strippkolonne 20 wird daraus wieder ein $C_1$-freies Kondensat und ein leichtes Kopfprodukt gewonnen. Dazu ist diese Kolonne mit elf theoretischen Böden versehen und arbeitet bei Temperaturen zwischen 254 K am Kopf und 292 K im Sumpf. Als Sumpfprodukt fallen 10 434 Nm³/h $C_1$-freies Kondensat an, und 5 460 Nm³/h leichte Bestandteile werden aus dem eingespeisten Kondensat abgetrennt. Dazu ist es nötig, im Sumpf über Wärmetauscher 22 stündlich 1,0 Gcal (4,2 GJ) Wärme zuzuführen, was wiederum über Waschwasser erfolgen kann.

Diese Verfahrensführung hat den Vorteil, dass keine der Strippkolonnen mehr mit vergleichsweise teurem Dampf im Sumpf beheizt werden muss, sondern dass die Verwendung von Waschwasser in den beiden verbleibenden Säulen möglich ist.

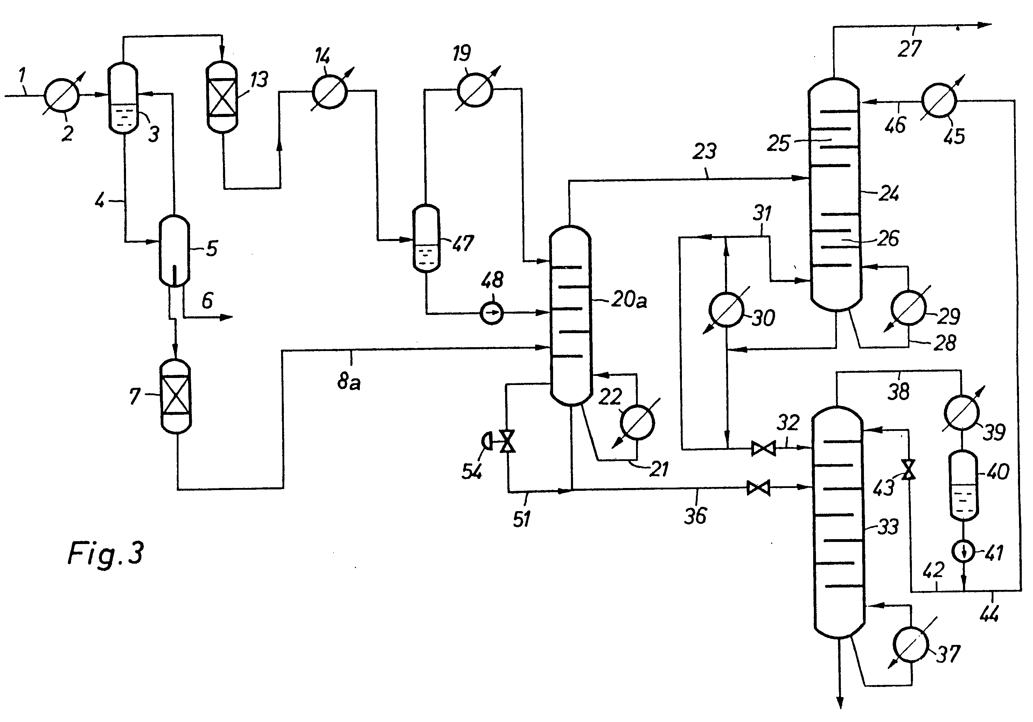

Im Verfahren der Figur 3 wird schliesslich nur noch eine Strippsäule 20a verwendet. Gegenüber dem Verfahren der Figur 2 ist die Strippsäule 15a durch einen Abscheider 47 ersetzt. Das in diesem Abscheider abgetrennte Kondensat wird über eine Pumpe 48 an geeigneter Stelle in die Strippkolonne 20a geführt. Die gasförmige Fraktion aus Abscheider 47 wird nach weiterer Kühlung im Wärmetauscher 19, bei der 1,5 Gcal/h (6,3 GJ/h) abgeführt werden, in die Strippkolonne 20a geleitet. Desweiteren wird über Leitung 8b auch das bei der ersten Kondensationsstufe anfallende Kondensat in einen unteren Bereich der Strippkolonne 20a geführt.

Die Strippkolonne ist bei diesem Ausführungsbeispiel mit elf theoretischen Böden versehen und wird bei einem Druck von 33,6 bar am Kopf und 33,7 bar im Sumpf betrieben. Die Kopftemperatur beträgt 250 K, die Sumpftemperatur 310 K. Bei einem Rücklaufverhältnis von 1,54 werden 28 876 Nm³/h $C_1$-freies Sumpfprodukt gebildet, das über Leitung 36 der Rektifiziersäule 33 zugeleitet wird. Der Wärmetauscher 22 wird mit 2,2 Gcal/h (9,2 GJ/h) beheizt. Aus den der Strippkolonne 20a aufgegebenen Kondensaten werden 8 995 Nm³/h gasförmige Bestandteile abgetrennt und gemeinsam mit dem nichtkondensierten Gasgemisch über Leitung 33 der Rektifiziersäule 24 zugeführt.

Bei dieser Verfahrensführung ist es erforderlich, für die Abtrennung der $C_2$-Kohlenwasserstoffe in der Rektifiziersäule 33 im Sumpf mit 4,3 Gcal/h (18,0 GJ/h) zu heizen. Für die Kondensation des Kopfprodukts sind im Wärmetauscher 39 3,7 Gcal/h (15,5 GJ/h) erforderlich.

In allen Beispielen wurde von einem Gasgemisch ausgegangen, das folgende Zusammensetzung aufweist (wasserfrei):

| | |
|---|---|
| $H_2$ | 1,30 Gew.-% |
| CO | 0,13 Gew.-% |
| $CH_4$ | 24,39 Gew.-% |
| $C_2H_2$ | 0,62 Gew.-% |
| $C_2H_4$ | 37,67 Gew.-% |
| $C_2H_6$ | 8,42 Gew.-% |
| $C_3H_4$ | 0,90 Gew.-% |
| $C_3H_6$ | 14,98 Gew.-% |
| $C_3H_8$ | 0,47 Gew.-% |
| $C_{4+}$ | 11,12 Gew.-% |

Der bereits kondensiert vorliegende Anteil hat folgende Zusammensetzung:

| | |
|---|---|
| $H_2$ | 0,03 Gew.-% |
| $CH_4$ | 2,14 Gew.-% |
| $C_2H_2$ | 0,20 Gew.-% |
| $C_2H_4$ | 10,34 Gew.-% |
| $C_2H_6$ | 3,01 Gew.-% |
| $C_3H_4$ | 0,80 Gew.-% |
| $C_3H_6$ | 13,27 Gew.-% |
| $C_3H_8$ | 0,48 Gew.-% |
| $C_{4+}$ | 69,73 Gew.-% |

Der Wassergehalt des Gases liegt bei 0,11 Gew.-%, der des kondensierten Anteils bei 1,54 Gew.-%

**Patentansprüche**

1. Verfahren zum Zerlegen eines im wesentlichen aus Kohlenwasserstoffen bestehenden Gasgemisches, bei dem durch ein- oder mehrstufige partielle Kondensation Teile des Gasgemisches verflüssigt werden, wobei die dabei gebildeten flüssigen Fraktionen in einer ersten Rektifiziersäule weiter zerlegt werden und wobei nach der letzten Stufe der partiellen Kondensation die schwer flüchtigen Bestandteile aus der verbliebenen gasförmigen Fraktion durch Rektifikation in einer zweiten Rektifiziersäule entfernt werden, dadurch gekennzeichnet, dass von den flüssigen Fraktionen vor deren Einspeisung in die erste Rektifiziersäule die am leichtesten flüchtigen Bestandteile abgestrippt werden.

2. Verfahren nach Anspruch 1, dadurch gekennzeichnet, dass die zweite Rektifiziersäule einen Rektifizierteil und einen Strippteil aufweist, wobei im Rektifizierteil die schwer flüchtigen Bestandteile aus der gasförmigen Fraktion abgetrennt und im Strippteil die dabei in Lösung gegangenen am leichtesten flüchtigen Bestandteile zumindest teilweise wieder abgestrippt werden.

3. Verfahren nach Anspruch 2, dadurch gekennzeichnet, dass das Sumpfprodukt der zweiten Rektifiziersäule der ersten Rektifiziersäule zur weiteren Zerlegung aufgegeben wird.

4. Verfahren nach einem der Ansprüche 1 bis 3, dadurch gekennzeichnet, dass die abgestrippten Bestandteile der flüssigen Fraktionen bzw. des Sumpfprodukts der zweiten Rektifiziersäule wieder den jeweiligen gasförmigen Fraktionen zugeführt werden.

5. Verfahren nach einem der Ansprüche 1 bis 4, dadurch gekennzeichnet, dass in der ersten Rektifiziersäule von den flüssigen Fraktionen die noch gelösten leicht flüchtigen Bestandteile abgetrennt werden, dass das Kopfprodukt der ersten Rektifiziersäule verflüssigt und zum einen Teil der ersten und zum anderen Teil dem Rektifizierteil der zweiten Rektifiziersäule als Rücklaufflüssigkeit aufgegeben wird.

6. Verfahren nach einem der Ansprüche 1 bis 5 zum Zerlegen eines Kohlenwasserstoffgemisches in eine $C_{2-}$- und eine $C_{3+}$-Fraktion, dadurch gekennzeichnet, dass aus den flüssigen Fraktionen die leichter als $C_2$-Kohlenwasserstoffe flüchtigen Bestandteile und in der ersten Rektifiziersäule die $C_2$-Kohlenwasserstoffe entfernt werden, und dass im Rektifizierteil der zweiten Rektifiziersäule die schwerer als $C_2$-Kohlenwasserstoffe flüchtigen Bestandtele aus der gasförmigen Fraktion abgetrennt werden.

7. Verfahren nach einem der Ansprüche 1 bis 5 zum Zerlegen eines Kohlenwasserstoffgemisches in eine $C_{1-}$- und eine $C_{2+}$-Fraktion, dadurch gekennzeichnet, dass aus den flüssigen Fraktionen die leichter als $C_1$-Kohlenwasserstoffe flüchtigen Bestandteile und in der ersten Rektifiziersäule die $C_1$-Kohlenwasserstoffe entfernt werden, und dass im Rektifizierteil der zweiten Rektifiziersäule die schwerer als $C_1$-Kohlenwasserstoffe flüchtigen Bestandteile aus der gasförmigen Fraktion abgetrennt werden.

### Claims

1. A process for separating a gas mixture consisting basically of hydrocarbons, wherein parts of the gas mixture are liquefied by single-stage or multi-stage partial condensation, wherein the liquid fractions so formed are further separated in a first rectification column, and wherein, after the last stage of the partial condensation, the least volatile components are removed from the remaining gaseous fraction by rectification in a second rectification column, characterised in that the most readily volatile components are stripped from the liquid fractions before they are fed into the first rectification column.

2. A process as claimed in Claim 1, characterised in that the second rectification column comprises a rectifying part and a stripping part, and wherein the least volatile components of the gaseous fraction are separated in the rectifying part, and the most readily volatile components which have thereby formed a solution, are at least partially stripped off again in the stripping part.

3. A process as claimed in Claim 2, characterised in that the sump product of the second rectification column is fed to the first rectification column for further separation.

4. A process as claimed in one of Claims 1 to 3, characterised in that the components stripped from the liquid fractions and the sump product of the second rectification column are respectively supplied to the respective gaseous fractions.

5. A process as claimed in one of Claims 1 to 4, characterised in that in the first rectification column, the readily volatile components still in solution, are stripped from the liquid fractions, and that the head product of the first rectification column is condensed and is partially fed to the first rectification column and partially to the rectifying part of the second rectification column as reflux liquid.

6. A process as claimed in one of Claims 1 to 5

for the separation of a hydrocarbon mixture into a $C_{2-}$-fraction and a $C_{3+}$-fraction characterised in that those components which are more readily volatile than $C_2$-hydrocarbons are removed from the liquid fractions and the $C_2$-hydrocarbons are removed in the first rectification column, and that those components of the gaseous fraction which are less readily volatile than the $C_2$-hydrocarbons are separated in the rectifying part of the second rectification column.

7. A process as claimed in one of Claims 1 to 5, for the separation of a hydrocarbon mixture into a $C_{1-}$-fraction and a $C_{2+}$-fraction, characterised in that those components which are more readily volatile than $C_1$-hydrocarbons are removed from the liquid fractions and $C_1$-hydrocarbons are removed in the first rectification column, and that those components of the gaseous fraction which are less readily volatile than $C_1$-hydrocarbons are separated in the rectifying part of the second rectification column.

### Revendications

1. Procédé de fractionnement d'un mélange gazeux essentiellement constitué d'hydrocarbures, dans lequel on liquéfie une partie du mélange par une condensation en une ou plusieurs étapes, on fractionne encore les fractions liquides dans une première colonne de distillation et, après la dernière étape de condensation partielle, on élimine les constituants peu volatils de la fraction gazeuse restante dans une deuxième colonne de distillation, caractérisé en ce que l'on extrait les constituants les plus volatils de la fraction liquide avant d'introduire celle-ci dans la première colonne de distillation.

2. Procédé selon la revendicatin 1, caractérisé en ce que la deuxième colonne de distillation comporte une zone de rectification et une zone d'extraction, en ce que l'on sépare les constituants peu volatils de la fraction gazeuse dans la zone de rectification et en ce que, dans la zone d'extraction, on extrait au moins en partie les constituants les plus volatils passés en solution lors de cette extraction.

3. Procédé selon la revendication 2, caractérisé en ce que l'on amène le produit de cuve de la seconde colonne de distillation dans la première colonne de distillation afin de poursuivre le fractionnement.

4. Procédé selon l'une quelconque des revendications 1 à 3, caractérisé en ce que les constituants extraits de la fraction liquide ou du produit de cuve de la deuxième colonne de distillation sont ajoutés de nouveau dans les fractions gazeuses correspondantes.

5. Procédé selon l'une quelconque des revendications 1 à 4, caractérisé en ce que, dans la première colonne de distillation, on sépare les constituants volatils encore en solution de la fraction liquide, en ce qu'on liquéfie le produit de tête de la première colonne de distillation et en ce qu'on en introduit une partie dans la première colonne de distillation et une autre partie dans la partie de distillation de la deuxième colonne de distillation, en tant que liquide de reflux.

6. Procédé selon l'une quelconque des revendications 1 à 5, pour fractionner un mélange d'hydrocarbures en une fraction en $C_{2-}$ et une fraction en $C_{3+}$, caractérisé en ce qu'on retire de la fraction liquide les constituants plus volatils que les hydrocarbures en $C_2$, cependant qu'on en retire les hydrocarbures en $C_2$ dans la première colonne de distillation, et en ce qu'on sépare de la fraction gazeuse les constituants moins volatils que les hydrocarbures en $C_2$ dans la zone de rectification de la deuxième colonne de distillation.

7. Procédé selon l'une quelconque des revendications 1 à 5 pour fractionner un mélange d'hydrocarbures en des fractions en $C_{1-}$ et $C_{2+}$, caractérisé en ce que l'on retire de la fraction liquide les constituants plus volatils que les hydrocarbures en $C_1$, cependant qu'on en retire les hydrocarbures en $C_1$ dans la première colonne de distillation et en ce que, dans la zone de rectification de la deuxième colonne de distillation, on sépare de la fraction gazeuse les constituants moins volatils que les hydrocarbures en $C_1$.

Fig. 1

Fig. 2

Fig.3

0010 223